# EUROPÄISCHE PATENTSCHRIFT

(11) **EP 0 720 832 B1**
(45) Veröffentlichungstag und Bekanntmachung des Hinweises auf die Patenterteilung: **14.04.1999**
(21) Anmeldenummer: 95100153.6
(22) Anmeldetag: 07.01.1995
(51) Int. Cl.: A61B 10/00

(54) **Testpflaster zur epicutanen Testung**
Epicutaneous test plaster
Sparadrap pour test épicutané

(43) Veröffentlichungstag der Anmeldung: 10.07.1996
(73) Patentinhaber: Siewert, Ronald-R., 40591 Düsseldorf (DE)
(72) Erfinder: Siewert, Ronald-R., D-40885 Ratingen (DE); Schmoll, Manfred Dr. med., D-26125 Oldenburg (DE)
(74) Vertreter: Meyers, Hans-Wilhelm, Dr.Dipl.-Chem.

(56) Entgegenhaltungen:
- WO-A-81/00199
- WO-A-91/01705
- WO-A-92/01421
- GB-A- 2 052 993
- US-A- 3 212 495

## Beschreibung

Gegenstand der vorliegenden Erfindung ist ein Testpflaster zur epicutanen Hauttestung gemäß Oberbegriff des Anspruchs 1.

Die DE-PS 24 20 345 beschreibt eine Hauttestdeckung zur Untersuchung der Wirkung eines Stoffes auf die menschliche Haut. Dabei befinden sich die zu testenden Substanzen in einem Abdeckungskörper, welcher einstückig aus einem Plattenteil und einem dessen Umfangsrand begrenzenden, mit der Haut in Berührung tretenden geraden Teil (Kragenteil) besteht. Der Andeckungskörper wird mit einem Pflaster zur Befestigung auf der Haut versehen. Der Kragenteil ist dabei in Richtung der Hautoberfläche abgewinkelt und die zur abdichtenden Berührung mit der Haut dienende Kante ist abgerundet.

Das deutsche Gebrauchsmuster G 84 19 329 U1 betrifft ein Hauttestpflaster, das aus einer Befestigungsunterlage und einem oder mehreren daran befestigten Testzentren besteht. Die Testzentren sind an ihrer Unterseite an der mit einem Klebstoff überzogenen Unterlage befestigt.

Die WO 92/01421, die als Basis für den Oberbegriff des Anspruchs 1 verwendet ist, betrifft ein Testpflaster mit quadratischen Testkammern, die auf einem Klebeband angeordnet sind und wobei die klebenden Stellen mit einer Schutzfolie abgedeckt sind. Die Schutzfolie weist Aussparungen an den Stellen auf, an denen die Testkammern auf der Klebefolie befestigt sind.

Die DE 38 10 658 betrifft ein Epicutan-Testpflaster, mit mindestens einer auf einer Trägerfolie angeordneten Wirkstoffaufnahmeeinrichtung, wobei die Trägerfolie ein für flüssiges Wasser dichtes, aber für Wasserdampf durchlässiges, hochelastisches Polymermaterial ist und an der der Haut abgewandten Fläche mit einer mindestens die Fläche der Trägerfolie abdeckenden Stützfolie ganzflächig oder an Teilflächenabschnitten lösbar verbunden ist. Die Wirkstoffaufnahmeeinrichtungen sind kreisrund ausgestaltet.

Zur Vorbereitung einer Testreihe werden üblicherweise die Testkammern mit Testallergenen befüllt. Diese Testallergene werden teilweise aus flüssiger Phase in die entsprechenden Kammern, die am Boden mit einem Vlies ausgestattet sein können, gefüllt und dann mit der Befüllseite nach unten auf die Haut des Probanden geklebt. Üblicherweise sind die Testpflaster mit einstückigen Deckfolien ausgestattet, die vor dem Befüllen vollständig entfernt werden müssen. Dabei kommt jedoch das mit der Befüllung der Testkammern beschäftigte Personal oft mit den Händen an klebende Bereiche der Folie, so daß sich das Testpflaster an die Hand oder Arbeitsschutzkleidung des Laborpersonals anheftet und eine sachgerechte Befüllung der Testkammern erschwert. Das Entfernen des anhaftenden Pflasters ist für den entsprechenden Mitarbeiter lästig und zeitintensiv. Dies kann sogar zum Verlust des Testpflaster führen, was dann mit nicht zu vernachlässigenden Kosten verbunden sein kann.

Das in der WO 92/01421 beschriebene Testpflaster kann befüllt werden, obwohl die die Klebeseite abdeckende Folie noch am Testpflaster verbleibt. Dabei wirkt sich jedoch nachteilig aus, daß die Testkammern geöffnet sind und leicht verschmutzen können oder bei unsachgemäßer Ablagerung in Mitleidenschaft gezogen werden können. In der WO 92/01421 wird daher in einer besonderen Ausführungsform auch darauf abgestellt, daß das beschriebene Testpflaster in Schutzfolie eingeschweißt ist. Dies führt dann aber zu einem Testpflaster, das nachteilig ist, weil zwei Folien zum Schutz der klebenden Bereiche bzw. der Kammern verwendet werden müssen.

Das der vorliegenden Erfindung zugrundeliegende Problem besteht mithin darin, die genannten Nachteile des Standes der Technik zu vermeiden und ein Testpflaster bereitzustellen, das zum einen die Kammern zur Aufnahme der Testallergene hinreichend vor Beeinträchtigungen schützt, andererseits aber ein ergonomisch sinnvolles Befüllen der Kammern erlaubt und womit eine exakten Positionierung zu erreichen ist.

Das der Erfindung zugrundeliegende technische Problem wird gelöst durch ein Testpflaster zur epicutanen Testung mit den Merkmalen des Anspruchs 1. Die Unteransprüche betreffen bevorzugte Ausführungsformen des erfindungsgemäßen Testpflasters.

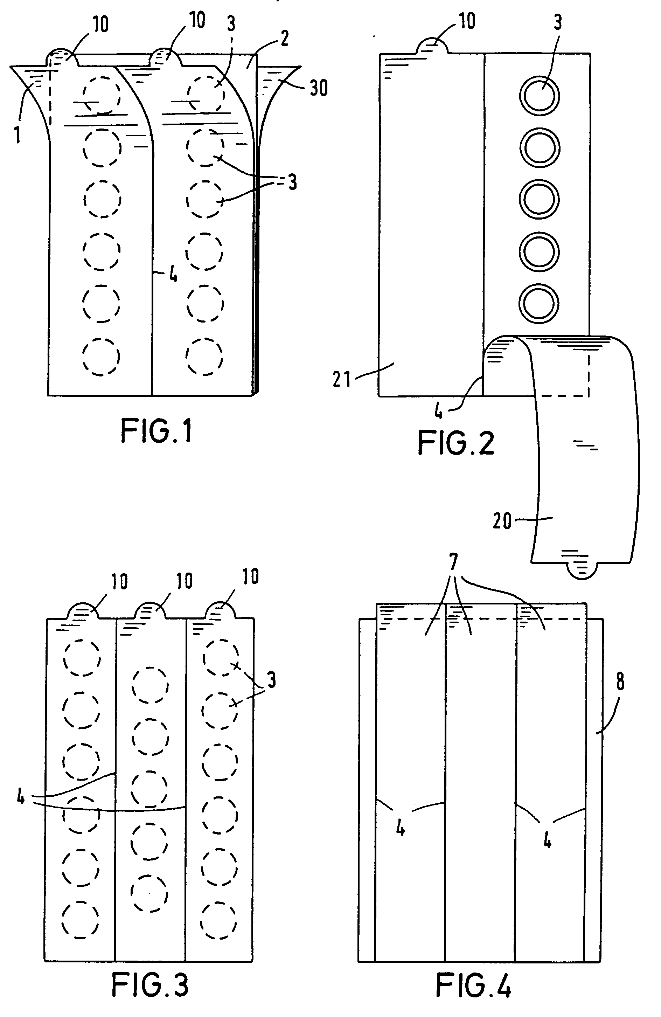

Die Figur 1 zeigt eine bevorzugte Ausführungsform des erfindungsgemäßen Testpflasters, bei der die Testkammern in einer zweireihigen Anordnung auf der klebende Seite der Klebefolie 2 befestigt sind. Die Deckfolie 1 ist im Bereich zwischen den Reihen, die durch die Testkammern 3 gebildet werden, längs der Linie 4 geteilt. Diese Linie kann durch Stanzung oder Vorstanzung der Deckfolie 1, Perforation der Deckfolie 1 oder durch einen Aufreißstreifen gebildet werden.

Die Deckfolie 1 wurde vorzugsweise einer Behandlung, wie Silikonisierung, unterzogen, die ihre Ablösung von der klebenden Seite der die Kammern 3 tragenden Folie 2 ermöglicht.

Die Figur 2 zeigt die Situation nach dem Abziehen des rechten Streifens 20 der Deckfolie 1 mit den darunterliegenden Testkammern 3. Diese können von der damit beschäftigten Person befüllt werden, wobei beispielsweise durch Auflegen der linken Hand auf den linken Streifen 21 der Deckfolie 1 das Pflaster fixiert werden kann, ohne daß die das Testpflaster fixierende Hand mit den klebenden Flächen der Klebefolie 2 in Berührung kommt.

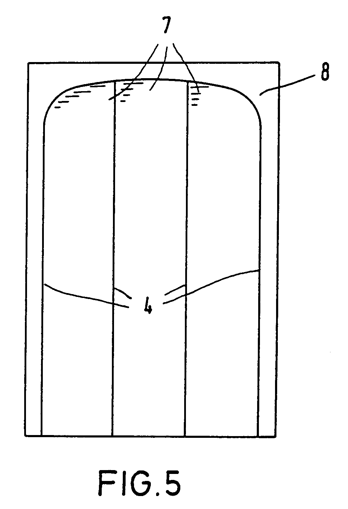

Die Figuren 3 bis 8 zeigen weitere bevorzugte Anordnungen des erfindungsgemäßen Testpflasters.

Die Figur 3 betrifft eine Anordnung, bei der drei Reihen von Testkammern auf der klebenden Seite der Klebefolie angebracht sind. Die Figur 4 zeigt eine Anordnung, bei der nach Abziehen der die Kammern bedeckenden Streifen 7 am Rand noch Streifen 8 verbleiben, die als nicht klebende Bereiche beim Anlegen des Pflasters Hilfestellung leisten und erst unmittelbar vor dem Anlegen des Pflasters am Patienten abgezogen werden, um eine ganzflächige Klebung zu gewährleisten. Diese Ausführungsform ist in Figur 5 in anderer Ausführung dargestellt. Der Rand 8 bildet dabei einen umlaufenden U-förmigen Bereich. Figur 6 zeigt die Rückansicht des erfindungsgemäßen Pflasters mit Trägerfolie. Die Figuren 7 und 8 zeigen die Testkammer 3 im Detail.

Vorzugsweise ist an den getrennt und unabhängig voneinander abziehbaren Bereichen eine Greifhilfe 10 angeordnet, mit der das Abziehen erleichtert wird.

Die Figur 1 zeigt eine bevorzugte Auführungsform des erfindungsgemäßen Testpflasters. Die Klebefolie 2 des erfindungsgemäßen Pflasters trägt vorzugsweise ovale Kammern 3 zur Aufnahme der auf der Haut zu testenden Stoffe. Die Folie 2 ist vorzugsweise eine für Luft und Wasserdampf durchlässige, transparente Kunststoffolie, die auf einer Seite mit einem hautverträglichen Haftkleber versehen ist. Auf dieser Seite der Folie 2 sind die Kammern 3 zur Aufnahme der zu testenden Stoffe angeordnet.

Die Klebefolie 2 des erfindungsgemäßen Testpflasters besteht vorzugsweise aus Polyurethan mit einer Stärke von 15 bis 35 µm. Vorzugsweise ist die Klebefolie 2 hochelastisch und dehnbar bis 700 %. Als besonders bevorzugt zum Einsatz im erfindungsgemäßen Pflaster hat sich eine im klinischen Bereich seit Jahren bewährte Polyurethanfolie, die unter der Bezeichnung Tegaderm® der Firma 3M, Minnesota, USA erhältlich ist, herausgestellt.

Der auf der Klebeseite der Klebefolie 2 des erfindungsgemäßen Testpflasters angeordnete Haftkleber ist vorzugsweise ein hypoallergener Haftklebstoff auf Acrylatbasis, der aufgrund seiner Zusammensetzung gleichzeitig eine gute Haftfähigkeit z.B. auf fettiger Haut gewährleistet und nach Abschluß der Testphase ein Abziehen des Pflasters ohne Schmerzen ermöglicht. Der Acrylatkleber sollte zudem vorzugsweise nur geringe Wasserlöslichkeit aufweisen, um ein Ablösen des Heftpflasters von der Haut z.B. beim Schwitzen oder beim Baden, Duschen oder Saunen zu vermeiden. Das erfindungsgemäße Testpflaster ermöglicht aufgrund seiner zwar wasserdampfdurchlässigen aber wasserabweisenden Eigenschaften dem Patienten hin und wieder Aktivitäten wie Duschen, Schwimmen etc, ohne den Test erheblich einzuschränken.

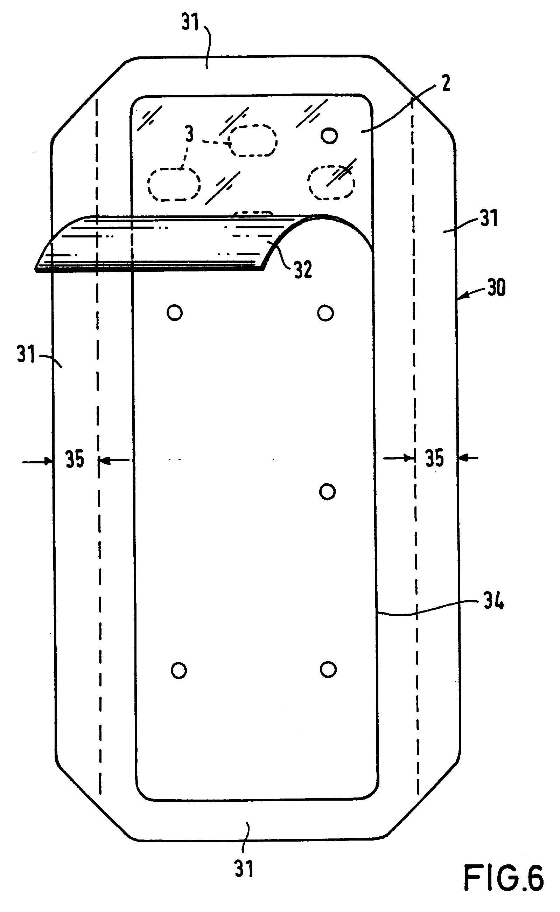

Das erfindungsgemäße Testpflaster weist vorzugsweise auf der nicht mit Haftkleber versehenen Seite der Klebefolie 2 eine Trägerfolie 30 auf (Figur 6). Vorzugsweise ist die Trägerfolie 30 mehrteilig ausgestaltet. Die Trägerfolie 30 des erfindungsgemäßen Testpflasters kann dabei durch Stanzung 34 in einem Bereich 31, der den Rand in einer gewissen Breite umfaßt, und dem mittleren Teil 32 getrennt sein. Nach dem Befüllen der ovalen Kammern 3 mit den Testsubstanzen wird zum Gebrauch die Deckfolie 1 abgezogen. Der die Klebseite der Klebefolie 2 überragende Teil 35 der Trägerfolie 30 dient zum sicheren Fassen des Testpflasters sowie als Greifhilfe zur erleichterten Trennung von Deckfolie 1 und Trägerfolie 30. Danach kann der mittlere Teil 32 der Trägerfolie 30 abgezogen werden, so daß der Rahmen 31 auf der nicht mit Haftkleber versehenen Seite der Klebefolie 2 verbleibt. Das durch das Abziehen des mittleren Teils 32 der Trägerfolie 30 entstehende Fenster ermöglicht die Sicht auf die Kammern 3 zur exakten Positionierung des gesamten Pflasters. Nachdem das erfindungsgemäße Testpflaster mit der klebenden Seite der Klebefolie 2 auf der Haut des Patienten angeordnet ist, kann der verbliebene Rest der Trägerfolie 30, der Rahmen 31, von der Oberseite der Klebefolie 2 abgezogen werden.

Durch gegebenenfalls in der Klebefolie 2, der Trägerfolie 30 und der Deckfolie 1 befindliche vorgestanzte Löcher kann eine Markierung des Testpflasters auf der Haut erfolgen.

In einer besonders bevorzugten Ausführungsform ist die ovale Kammer 3 aus einem transparenten Material gefertigt. Die transparente ovale Kammer 3 in Verbindung mit der transparenten Klebefolie 2 hat den Vorteil, daß eine permanente Beobachtung der unter der ovalen Kammer 3 befindlichen Haut, die den in der Kammer befindlichen Allergenen ausgesetzt ist, gewährleistet ist.

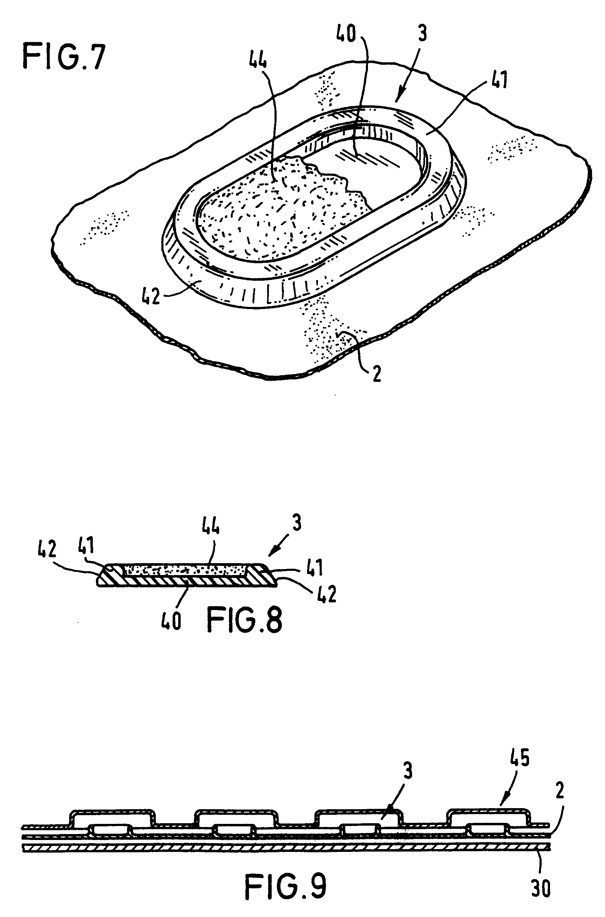

Die ovale Kammer 3 (Fig. 7 und 8) ist vorzugsweise einstückig ausgebildet, wobei das innere Lumen der Testkammer durch einen Boden 40 und rings umherlaufender Wandung 41 zur Erzielung eines okklusiven Verschlusses zwischen Testkammer und Hautareal gebildet wird. Die innere Seite der Wandung 41 bildet mit dem Boden 40 vorzugsweise einen rechten Winkel. Die obere Begrenzung der Wandung 41 ist parallel zur Bodenfläche ausgebildet. Die äußere Wandung 42 fällt vorzugsweise nicht rechtwinklig zur Bodenplatte hin ab, sondern weist vorzugsweise einen Böschungswinkel (Winkel zwischen Boden 40 und äußerer Wandung 41) < 90° auf.

Die ovale Kammer 3 weist vorzugsweise eine Innenfläche von 50 bis 70 mm² auf. Die Abstände zwischen den einzelnen Testkammern sind so groß gewählt, daß es nicht zu einander beeinflussenden Reaktionen der Testallergene auf der Haut kommt.

Im inneren Lumen der ovalen Kammern 3 befindet sich ein Vlies 44 oder eine andere saugfähige Unterlage fest angeordnet, um das Auslaufen der Testflüssigkeit aus den ovalen Kammern 3 zu verhindern. Besonders bevorzugt ist die Verwendung von transparenten Vliesen, die im Zusammenspiel mit der Transparenz der Kammern und der Klebefolie 2 ebenfalls eine dauernde Beobachtung der mit Allergenen kontaktierten Stelle erlauben.

Vorzugsweise sind auf der klebenden Seite der Klebefolie 2 so viele ovale Kammern 3 angeordnet, wie der der europäischen Standardtestreihe entsprechen. Vorteilhaft an den ovalen Kammern 3 des erfindungsgemäßen Testpflasters ist deren Eigenschaft, durch die ovale Form eine bessere Unterscheidung allergischer Reaktionen von anderen Störungen der Hautfunktion zu ermöglichen. Die ovalen Kammern 3 weisen keine scharfen Winkel auf, welche Abdrücke auf der Haut hinterlassen und den Patienten beim Liegen Schmerzen bereiten könnten. Durch die Verwendung von Polyethylen zur Herstellung der ovalen Kammern 3 werden Interaktionen und Unverträglichkeiten bei Metalltestungen zwischen dem Kammermaterial und den Testallergenen vermieden, die auftreten können, wenn Kammern aus Metall verwendet werden.

Die ovalen Kammern 3 werden vorzugsweise mit ihrer Bodenplatte 40 direkt auf der klebenden Seite der Klebefolie 2 angeordnet. Dabei ist es vorteilhaft die Unterseite der Kammern, die mit dem Kleber der Klebefolie 2 in Berührung kommen, vorher durch Coronaentladung oder chemische Verfahren zu aktivieren. Dies führt zu einer intensiven Haftung der ovalen Kammern 3 auf der klebenden Seite der Klebefolie 2.

Die Handhabung des Testpflasters wird im folgenden näher beschrieben. Das erfindungsgemäße Testpflaster wird zur Vorbereitung der epicutanen Hauttestungen so plaziert, daß die Deckfolie 1 nach oben zu liegen kommt. Der Bereich 20 wird abgezogen. Danach werden die einzelnen Testallergene in die ovalen Kammern 3 gegeben, die durch Abziehen des Bereiches 20 zugänglich geworden sind. Es empfiehlt sich dabei, eine bestimmte Reihenfolge der Füllung einzuhalten. Beispielsweise kann die Beschickung mit der rechts oben liegenden ovalen Kammer 3 beginnen. Es empfiehlt sich, die ovalen Kammern 3 von oben nach unten zu befüllen. Dann wird der Bereich 21 abgezogen und wie oben beschrieben befüllt.

Die Trägerfolie 30 überragt die Klebefolie 2, so daß dadurch ein nicht klebender Bereich vorhanden ist, welcher die klebende Seite der Klebefolie 2 in Form eines Rahmens umgibt. An diesem Rahmen kann das Testpflaster gehalten werden, ohne die klebenden Bereiche zu berühren. Nachdem die Testallergene in die ovalen Kammern 3 abgefüllt wurden und gegebenenfalls von dem in den ovalen Kammern 3 angeordneten Vlies aufgesaugt worden sind, kann das Testpflaster umgedreht werden und der hintere Bereich 32 der vorgestanzten Trägerfolie 30 abgezogen werden. Es kommen dann die Unterseiten der ovalen Kammern 3 zum Vorschein. Danach wird das Pflaster mit der klebenden Seite auf die Haut des Patienten aufgebracht. Dabei sollte das Testpflaster auf die vorgespannte Haut, zum Beispiel dem gekrümmten Rücken, aufgetragen werden. Durch diese Maßnahme wird die Haftfähigkeit des Pflasters gesteigert und der Patient erhält einen größeren Bewegungsspielraum. Wenn das Testpflaster auf der Haut fixiert ist, wird der äußere Bereich 31 der Deckfolie ebenfalls abgezogen. Gegebenenfalls kann durch die in der Trägerfolie angebrachten gestanzten Markierungslöcher eine Markierung auf der Haut angebracht werden.

Das erfindungsgemäße Testpflaster läßt sich durch einfaches Zerschneiden in kleinere Formate umwandeln, sofern die ovalen Kammern nicht alle benötigt werden, wenn nicht alle Testallergene der europäischen Standardreihe verwendet werden.

Die Figur 9 zeigt einen Querschnitt durch eine Ausführungsform des erfindungsgemäßen Pflasters, bei dem eine zusätzliche Abdeckfolie 45 aufgebracht ist. Diese zusätzliche Abdeckfolie 45 besteht insbesondere aus einer transparenten Folie, die mit tiefgezogenen Erhebungen versehen ist. Die Erhebungen sind so angeordnet, daß sie über die Testkammern 3 gelegt werden können. Dies dient der Abdeckung bereits vorgefüllter Testpflaster, die dann stapelbar sind. In den entsprechenden Praxen oder Kliniken ist es üblich, daß das Laborpersonal Testpflaster vorzeitig (1 bis 3 Tage im voraus) befüllt, um bei Allergietests die Patienten schnell mit den Testpflastern zu versehen. Dies erspart den Patienten die Wartezeiten bei der Präparation der benötigten Testpflaster. Es ist aber bevorzugt, daß die Erhöhungen der zusätzlichen Abdeckfolie 45 deutlich größer und höher sind als die Testkammern auf dem Testpflaster.

Das erfindungsgemäße Testpflaster kann einzeln oder in Gruppen verpackt vertrieben werden. Grundsätzlich ist eine Sterilverpackung nicht erforderlich.

## Patentansprüche

1. Testpflaster für epicutane Testung mit einer selbstklebenden Folie, auf deren klebender Seite Kammern zur Aufnahme von Testallergenen angeordnet sind sowie einer die klebende Seite der Klebefolie abdeckende, abziehbare Deckfolie (1) die Deckfolie (1) mindestens zwei Bereiche (20, 21) umfaßt, die getrennt und unabhängig voneinander abziehbar sind, dadurch gekennzeichnet, daß die Trägerfolie (30), Klebefolie (2) und Deckfolie (1) mit durchgängigen Perforationen versehen sind, wodurch eine Markierung auf der Haut ermöglicht wird.

2. Testpflaster nach Anspruch 1, dadurch gekennzeichnet, daß die Kammern (3) zur Aufnahme von Testallergenen in mindestens zwei Reihen angeordnet sind.

3. Testpflaster nach Anspruch 2, dadurch gekennzeichnet, daß die Deckfolie (1) so viele Bereiche umfaßt, wie Reihen von Kammern zur Aufnahme von Testallergenen vorgesehen sind.

4. Testpflaster nach mindestens einem der Ansprüche 1 bis 3, dadurch gekennzeichnet, daß die Bereiche der Deckfolie (1), die unabhängig und getrennt voneinander abziehbar sind, durch Stanzung, Perforation oder Aufreißstreifen gebildet werden.

5. Testpflaster nach Anspruch 4, dadurch gekennzeichnet, daß die getrennt und unabhängig voneinander abziehbaren Bereiche der Deckfolie (1) mit Greifhilfen (10) ausgestattet sind.

6. Testpflaster nach Anspruch 5, dadurch gekennzeichnet, daß die Testkammern (3) oval ausgestaltet sind.

7. Testpflaster nach mindestens einem der Ansprüche 1 bis 6, dadurch gekennzeichnet, daß eine zusätzliche Abdeckfolie 45 mit tiefgezogenen Erhebungen vorgesehen ist, zum Starten der mit der zusätzlichen Deckfolie abgedeckten vorbefüllten Testpflaster.

## Claims

1. A test plaster for epicutaneous testing comprising a self-adhesive sheet with compartments for receiving test allergens provided on the adhesive side thereof, and a strippable cover sheet (1) covering the adhesive side of said adhesive sheet, said cover sheet (1) comprising at least two regions (20, 21) which can be separately and independently stripped, characterized in that the support sheet (30), adhesive sheet (2) and cover sheet (1) are provided with through perforations permitting a mark to be made on the skin.

2. The test plaster according to claim 1, characterized in that said compartments (3) for receiving test allergens are arranged in at least two rows.

3. The test plaster according to claim 2, characterized in that said cover sheet (1) comprises as many regions as rows of compartments for receiving test allergens are provided.

4. The test plaster according to at least one of claims 1 to 3, characterized in that said regions of the cover sheet (1) which can be separately and independently stripped are formed by punching, perforation or tear-open strips.

5. The test plaster according to claim 4, characterized in that said regions of the cover sheet (1) which can be separately and independently stripped are provided with gripping aids (10).

6. The test plaster according to claim 5, characterized in that said test compartments (3) have an oval design.

7. The test plaster according to at least one of claims 1 to 6, characterized in that an additional covering sheet 45 having deep-drawn protrusions is provided for starting the prefilled test plasters covered by said additional cover sheet.

## Revendications

1. Sparadrap d'essai pour test épicutané avec une feuille autocollante sur la face adhésive de laquelle sont disposés des compartiments destinés à recevoir des allergènes d'essai, ainsi qu'une feuille de recouvrement (1) pelable qui recouvre la face adhésive de la feuille collante, la feuille de recouvrement (1) comprenant au moins deux zones (20, 21) qui peuvent être enlevées par pelage séparément et indépendamment l'une de l'autre, caractérisé en ce que la feuille de support (30), la feuille collante (2) et la feuille de recouvrement (1) sont pourvues de perforations traversantes qui permettent un marquage sur la peau.

2. Sparadrap d'essai selon la revendication 1, caractérisé en ce que les compartiments (3) destinés à recevoir des allergènes d'essai sont disposés au moins sur deux rangées.

3. Sparadrap d'essai selon la revendication 2, caractérisé en ce que la feuille de recouvrement (1) comprend autant de zones qu'il est prévu de rangées de compartiments destinés à recevoir des allergènes d'essai.

4. Sparadrap d'essai selon au moins une des revendications 1 à 3, caractérisé en ce que les zones de la feuille de recouvrement (1), qui peuvent être enlevées par pelage séparément et indépendamment les unes des autres, sont formées par matriçage, par perforation ou par des languettes à déchirer.

5. Sparadrap d'essai selon la revendication 4, caractérisé en ce que les zones de la feuille de recouvrement (1), qui peuvent être enlevées par pelage séparément et indépendamment les unes des autres, sont pourvues de moyens auxiliaires de préhension (10).

6. Sparadrap d'essai selon la revendication 5, caractérisé en ce que les compartiments d'essai (3) sont de forme ovale.

7. Sparadrap d'essai selon au moins une des revendications 1 à 6, caractérisé en ce qu'il est prévu une feuille de recouvrement 45 supplémentaire avec des parties saillantes réalisées par emboutissage pour démarrer les sparadraps d'essai préalablement remplis et recouverts par la feuille de recouvrement supplémentaire.
